# EUROPEAN PATENT SPECIFICATION

(11) **EP 3 530 129 B1**
(45) Date of publication and mention of the grant of the patent: **07.04.2021**
(21) Application number: 17862415.1
(22) Date of filing: 17.10.2017
(51) Int. Cl.: A24F 40/485

(54) **SEPARATE AIR INLET DEVICE FOR A CIGARETTE SET**
GETRENNTE LUFTEINLASSVORRICHTUNG FÜR EIN ZIGARETTENSET
UN DISPOSITIF D'ENTRÉE D'AIR SÉPARÉ POUR UN ENSEMBLE DE CIGARETTE

(30) Priority: 18.10.2016 CN 201610906763
(43) Date of publication of application: 28.08.2019
(73) Proprietor: China Tobacco Yunnan Industrial Co., Ltd, Kunming, Yunnan 650231 (CN)
(72) Inventor: ZHENG, Xudong, Kunming Yunnan 650231 (CN); TANG, Jianguo, Kunming Yunnan 650231 (CN); ZENG, Xu, Kunming Yunnan 650231 (CN); WANG, Ru, Kunming Yunnan 650231 (CN); WANG, Chengya, Kunming Yunnan 650231 (CN); SHANG, Shanzhai, Kunming Yunnan 650231 (CN); LEI, Ping, Kunming Yunnan 650231 (CN); HAN, Jingmei, Kunming Yunnan 650231 (CN); LI, Zhiqiang, Kunming Yunnan 650231 (CN); YUAN, Dalin, Kunming Yunnan 650231 (CN); ZHAO, Liheng, Kunming Yunnan 650231 (CN); ZHANG, Jianrong, Kunming Yunnan 650231 (CN); CHEN, Yongkuan, Kunming Yunnan 650231 (CN); LUO, Hongyong, Kunming Yunnan 650231 (CN); FANG, Fengren, Kunming Yunnan 650231 (CN)
(74) Representative: Gille Hrabal Partnerschaftsgesellschaft mbB Patentanwälte
(86) International application number: PCT/CN2017/106532
(87) International publication number: WO 2018/072682

(56) References cited:
- WO-A1-2013/050934
- WO-A1-2015/120588
- WO-A1-2015/161556
- CN-A- 104 856 238
- CN-A- 105 455 198
- CN-A- 106 376 977
- CN-U- 202 262 413
- CN-U- 203 873 000
- CN-U- 203 873 000
- US-A1- 2014 196 733

## Description

### Technical Field

The present invention belongs to the field of electronic cigarette, specifically relates to a device with a separated gas inlet for cigarette set.

### Background

In the existing cigarette set for electronic cigarette, the traditional tobacco and controller switch share the same gas inlet channel, and the cigarette smoke passes through the hole on the controller switch. Since the smoke contains tarry matters, the tarry matters may attach on the hole, and with the increase of smoking cigarette, the tarry matters may accumulate to completely block the hole, causing a failure of the controller switch. WO 2015/120588 A1 discloses an electronic cigarette.

### Summary

In order to solve the above-mentioned problem, based on the existing structure with shared gas channel, the present invention provides a device with a separated gas inlet for cigarette set. Namely, air inlet channel for initiating a controller switch is separated from a smoke gas channel of a cigarette, avoiding the failure of the controller switch caused by tarry matters of traditional cigarette attaching on an initiating hole of the controller switch, and prolonging the service life of an electronic cigarette in cigarette set. A device with a separated gas inlet for cigarette set of the present invention includes:
a controller switch 2, wherein the controller switch is a cylindrical airflow sensor having two ends, and air can flow between the two ends;
a controller switch seat 3, wherein the controller switch seat is a cylinder-like structure with two ends, air can flow through each end, an inner cylinder is a controller switch hole 301 tightly attached to an outer wall of the controller switch 2, a wall perpendicular to an end of the controller switch seat 3 is provided with a U-shaped controller switch gas inlet channel 302 connecting an interior of the cylinder to an exterior of the cylinder, and a wall of the end is provided with an outer side wall groove 305, two grooves are provided along an axial direction of an outer side wall of the cylinder, namely, a first clamping guide groove 303 and a cigarette gas inlet channel 304, respectively;
a transparent light guide seat 4, wherein the transparent light guide seat is a cylinder-like structure closed in the middle, a second clamping guide groove 401 is provided along an axial direction of an inner side of an outer wall of the cylinder, a wall perpendicular to an end of the transparent light guide seat 4 is provided with a U-shaped controller switch gas inlet groove 403 connecting an interior of the cylinder to an exterior of the cylinder, and the end is provided with a controller switch matching a protruding wall 404 projecting outwards and tightly attached to the outer side wall groove 305, a closed central part in the middle of the cylinder is a controller switch hole plug 402, a first cigarette gas inlet hole 405 connected to the cigarette gas inlet channel 304 is provided between the controller switch hole plug 402 and the cylinder wall;
a filter holding seat 5, wherein the filter holding seat 5 is a cylinder-like structure with a closed end, a plurality of small holes provided on the closed end are second cigarette gas inlet holes 502, a clamping guide column 501 protruding along an axial direction of the closed end is in an interference fit respectively with the first clamping guide groove 303 and the second clamping guide groove 401, the filter holding seat 5 is sealed and in a clearance fit with another end of the transparent light guide seat 4, and the controller switch seat 3 is in a interference fit with the transparent light guide seat 4;
the second cigarette gas inlet holes 502 are respectively connected to the first cigarette gas inlet hole 405 and the cigarette gas inlet channel 304 on the controller switch seat 3 through a gap between the filter holding seat 5 and the transparent light guide seat 4;
an insulation fixing sleeve 6, wherein the insulation fixing sleeve is in a cylindrical-like shape, and sleeves outside the filter holding seat 5 in an interference fit;
a charging base 7, wherein the charging base is in a cylindrical-like shape, an upper portion of the charging base is sleeved outside the insulation fixing sleeve 6 in an interference fit, a lower portion of the charging base is sleeved inside a mouthpiece tube 1, a middle portion of the charging base is provided with a plurality of gas guide holes 71 connected to the controller switch gas inlet groove 403 and the controller switch gas inlet channel 302;
the air enters the controller switch gas inlet groove 403 and the controller switch gas inlet channel 302 from the gas guide holes 71, and then enters the controller switch 2, to initiate the electronic cigarette;
while the smoke gas enters the gap between the filter holding seat 5 and the transparent light guide seat 4 from the second cigarette gas inlet holes 502 to enter the first cigarette gas inlet hole 405, and then enters the cigarette gas inlet channel 304, and thus the mouthpiece tube 1.

Preferably, the lower portion of the charging base 7 is sleeved inside the mouthpiece tube 1 in an interference fit.

Preferably, the cigarette gas inlet channel 304 is connected to the mouthpiece tube 1.

The working principle of the device with the separated gas inlet for cigarette set of the present invention is as follows.

The controller switch 2 is installed in the controller switch seat 3, the controller switch scat 3 is in an interference fit with the transparent light guide scat 4, and the controller switch gas inlet groove 403 is connected to the controller switch gas inlet channel 302. The transparent light guide seat 4 is in a clearance fit with the filter holding seat 5 to make the second cigarette gas inlet holes 502 connected to the gap between the filter holding seat 5 and the transparent light guide seat 4, the first cigarette gas inlet hole 405, and the cigarette gas inlet channel 304. The clamping guide column 501 is in an interference fit respectively with the first clamping guide groove 303 and the second clamping guide groove 401. The insulation fixing sleeve 6 is sleeved outside the filter holding seat 5 in an interference fit. The charging base 7 is sleeved outside the insulation fixing sleeve 6 in an interference fit, and an outer side of another end of the charging base 7 is in an interference fit with an inner side of the mouthpiece tube 1 to make the gas guide holes 71 connected to the controller switch gas inlet groove 403 and the controller switch gas inlet channel 302.

The air enters the controller switch gas inlet groove 403 and the controller switch gas inlet channel 302 from the gas guide holes 71, and then enters the controller switch 2, to initiate the electronic cigarette.

While, the smoke gas enters the gap between the filter holding seat 5 and the transparent light guide seat 4 from the second cigarette gas inlet holes 502 to enter the first cigarette gas inlet hole 405, then enters the cigarette gas inlet channel 304, and thus the mouthpiece tube 1 for user to smoke.

The advantages of the present invention are as follows.

Based on the existing structure sharing the same gas channel, the present invention provides a device with a separated gas inlet for cigarette set. Namely, air inlet channel for air initiating a controller switch is separated from smoke gas channel for cigarette, avoiding the failure of the controller switch caused by tarry matters of traditional cigarette attaching on an initiating hole of the controller switch, and prolonging the service life of cigarette set.

### Brief Description of the Drawings

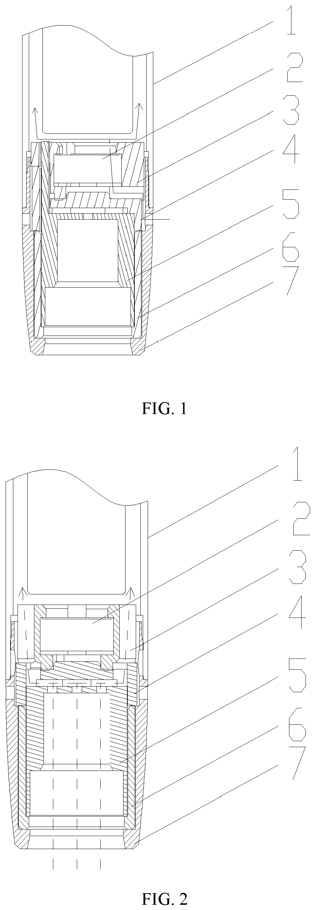

FIG. 1 is a schematic diagram showing a device with a separated gas inlet for cigarette set of the present invention in an assembled state and a flowing direction of gas initiating a controller switch;
FIG. 2 is a schematic diagram showing a device with a separated gas inlet for cigarette set of the present invention in an assembled state and a flowing direction of smoke gas;

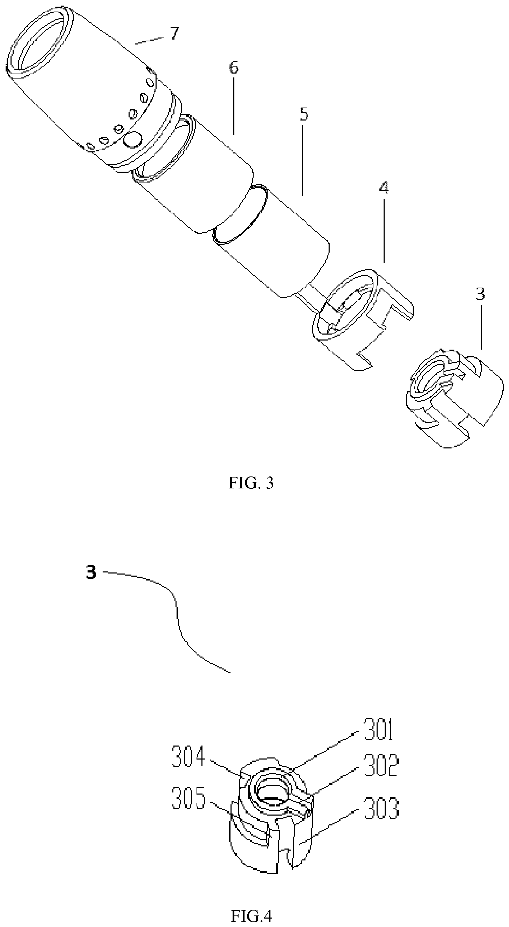

FIG. 3 is an exploded view of a device with a separated gas inlet for cigarette set of the present invention;
FIG. 4 is a schematic diagram of a controller switch seat of a device with a separated gas inlet for cigarette set of the present invention;

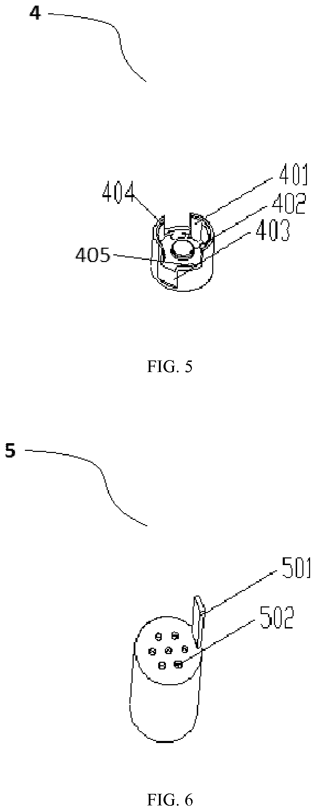

FIG. 5 is a schematic diagram of a transparent light guide scat of a device with a separated gas inlet for cigarette set of the present invention;
FIG. 6 is a schematic diagram of a filter holding seat of a device with a separated gas inlet for cigarette set of the present invention;

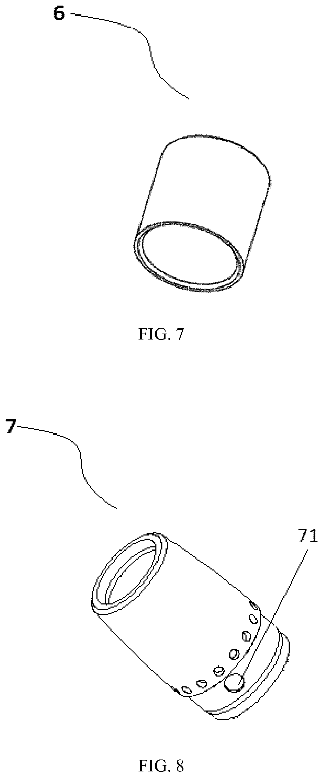

FIG. 7 is a schematic diagram of an insulation fixing sleeve of a device with a separated gas inlet for cigarette set of the present invention; and
FIG. 8 is a schematic diagram of a charging base of a device with a separated gas inlet for cigarette set of the present invention;

The reference designators in the drawings are described hereinafter. 1-mouthpiece tube; 2-controller switch; 3-controller switch seat; 301-controller switch hole; 302-controller switch gas inlet channel; 303-first clamping guide groove; 304-cigarette gas inlet channel; 305-outer side wall groove; 4-transparent light guide seat; 401-second clamping guide groove; 402-controller switch hole plug; 403-controller switch gas inlet groove; 404-controller switch matching protruding wall; 405-first cigarette gas inlet hole; 5-filter holding seat; 501-clamping guide column; 502-first cigarette gas inlet hole; 6-insulation fixing sleeve; 7-charging base.

### Detailed Description of the Embodiments

The controller switch 2 is installed in the controller switch seat 3, the controller switch seat 3 is in an interference fit with the transparent light guide seat 4, and the controller switch gas inlet groove 403 is connected to the controller switch gas inlet channel 302. The transparent light guide scat 4 is in a clearance fit with the filter holding seat 5 to make the second cigarette gas inlet holes 502 connected to the gap between the filter holding seat 5 and the transparent light guide seat 4, the first cigarette gas inlet hole 405, and the cigarette gas inlet channel 304. The clamping guide column 501 is in an interference fit respectively with the first clamping guide groove 303 and the second clamping guide groove 401. The insulation fixing sleeve 6 is sleeved outside the filter holding seat 5 in an interference fit. The charging base 7 is sleeved outside the insulation fixing sleeve 6 in an interference fit, and an outer side of another end of the charging base 7 is in an interference fit with an inner side of the mouthpiece tube 1 to make the gas guide holes 71 connected to the controller switch gas inlet groove 403 and the controller switch gas inlet channel 302.

The air enters the controller switch gas inlet groove 403 and the controller switch gas inlet channel 302 from the gas guide holes 71, and then enters the controller switch 2, to initiate the electronic cigarette.

The smoke gas enters the gap between the filter holding seat 5 and the transparent light guide seat 4 from the second cigarette gas inlet holes 502 to enter the first cigarette gas inlet hole 405, and then enters the cigarette gas inlet channel 304, and thus the mouthpiece tube 1 for user to smoke.

## Claims

1. A device with a separated gas inlet for cigarette set, comprising:
a controller switch (2), wherein the controller switch is a cylindrical airflow sensor having two ends, and air flows between the two ends;
a controller switch seat (3), wherein the controller switch seat is a cylinder-like structure with two ends, the air flows through each end, an inner cylinder is a controller switch hole (301) tightly attached to an outer wall of the controller switch (2), a wall perpendicular to an end of the controller switch seat (3) is provided with a U-shaped controller switch gas inlet channel (302) connecting an interior of the cylinder to an exterior of the cylinder, and a wall of the end is provided with an outer side wall groove (305), two grooves are provided along an axial direction of an outer side wall of the cylinder, namely, a first clamping guide groove (303) and a cigarette gas inlet channel (304), respectively;
a transparent light guide seat (4), wherein the transparent light guide seat is a cylinder-like structure closed in the middle, a second clamping guide groove (401) is provided along an axial direction of an inner side of an outer wall of the cylinder, a wall perpendicular to an end of the transparent light guide seat (4) is provided with a U-shaped controller switch gas inlet groove (403) connecting an interior of the cylinder to an exterior of the cylinder, and the end is provided with a controller switch matching a protruding wall (404) projecting outwards and tightly attached to the outer side wall groove (305), a closed central part at the middle of the cylinder is a controller switch hole plug (402), a first cigarette gas inlet hole (405) connected to the cigarette gas inlet channel (304) is provided between the controller switch hole plug (402) and the cylinder wall;
a filter holding seat (5), wherein the filter holding seat is a cylinder-like structure with a closed end, a plurality of small holes provided on the closed end are second cigarette gas inlet holes (502), a clamping guide column (501) protruding along an axial direction of the closed end is in an interference fit respectively with the first clamping guide groove (303) and the second clamping guide groove (401), the filter holding seat (5) is sealed and in a clearance fit with another end of the transparent light guide seat (4), and the controller switch seat (3) is in a interference fit with the transparent light guide seat (4);
the second cigarette gas inlet holes (502) are respectively connected to the first cigarette gas inlet hole (405) and the cigarette gas inlet channel (304) on the controller switch seat (3) through a gap between the filter holding seat (5) and the transparent light guide seat (4);
an insulation fixing sleeve (6), wherein the insulation fixing sleeve is in a cylindrical-like shape, and sleeves outside the filter holding seat (5) in an interference fit;
a charging base (7), wherein the charging base is in a cylindrical-like shape, an upper portion of the charging base is sleeved outside the insulation fixing sleeve (6) in an interference fit, a lower portion of the charging base is sleeved inside a mouthpiece tube (1), a middle portion of the charging base is provided with a plurality of gas guide holes (71) connected to the controller switch gas inlet groove (403) and the controller switch gas inlet channel (302);
air enters the controller switch gas inlet groove (403) and the controller switch gas inlet channel (302) from the gas guide holes (71), and then enters the controller switch (2), to initiate the electronic cigarette;
smoke gas enters the gap between the filter holding seat (5) and the transparent light guide scat (4) from the second cigarette gas inlet holes (502) to enter the first cigarette gas inlet hole (405), then enters the cigarette gas inlet channel (304), and thus the mouthpiece tube (1).

2. The device with the separated gas inlet for cigarette set of claim 1, wherein the lower portion of the charging base (7) is sleeved inside the mouthpiece tube (1) in an interference fit.

3. The device with the separated gas inlet for cigarette set of claim 1, wherein the cigarette gas inlet channel (304) is connected to the mouthpiece tube (1).

## Patentansprüche

1. Vorrichtung mit einem getrennten Gaseinlass für Zigarettenset, umfassend:
einen Steuerungsschalter (2), wobei der Steuerungsschalter ein zylindrischer Luftstromsensor mit zwei Enden ist und Luft zwischen den beiden Enden strömt;
einen Steuerungsschaltersitz (3), wobei der Steuerungsschaltersitz eine zylinderartige Struktur mit zwei Enden ist, die Luft durch jedes Ende strömt, ein innerer Zylinder ein fest an einer äußeren Wand des Steuerungsschalters (2) angebrachtes Steuerungsschalterloch (301) ist, eine senkrecht zu einem Ende des Steuerungsschaltersitzes (3) stehende Wand mit einem U-förmigen Steuerungsschalter-Gaseinlasskanal (302) versehen ist, der ein Inneres des Zylinders mit einem Äußeren des Zylinders verbindet, und eine Wand des Endes mit einer äußeren Seitenwandnut (305) versehen ist, zwei Nuten entlang einer axialen Richtung einer äußeren Seitenwand des Zylinders bereitgestellt sind, nämlich eine erste Klemmführungsnut (303) bzw. ein Zigarettengaseinlasskanal (304);
einen transparenten Lichtleitersitz (4), wobei der transparente Lichtleitersitz eine zylinderartige Struktur ist, die in der Mitte geschlossen ist, eine zweite Klemmführungsnut (401) entlang einer axialen Richtung einer inneren Seite einer äußeren Wand des Zylinders bereitgestellt ist, eine senkrecht zu einem Ende des transparenten Lichtleitersitzes (4) stehende Wand mit einer U-förmigen Steuerungsschalter-Gaseinlassnut (403) versehen ist, die ein Inneres des Zylinders mit einem Äußeren des Zylinders verbindet, und das Ende mit einem Steuerungsschalter versehen ist, der an eine vorstehende Wand (404) angepasst ist, die nach außen ragt und fest an der äußeren Seitenwandnut (305) angebracht ist, ein geschlossener Mittelteil in der Mitte des Zylinders ein Steuerungsschalter-Lochstopfen (402) ist, ein erstes Zigarettengaseinlassloch (405), das mit dem Zigarettengaseinlasskanal (304) verbunden ist, zwischen dem Steuerungsschalter-Lochstopfen (402) und der Zylinderwand bereitgestellt ist;
einen Filterhaltesitz (5), wobei der Filterhaltesitz eine zylinderartige Struktur mit einem geschlossenen Ende ist, eine Vielzahl kleiner Löcher, die an dem geschlossenen Ende bereitgestellt sind, zweite Zigarettengaseinlasslöcher (502) sind, eine Klemmführungssäule (501), die entlang einer axialen Richtung des geschlossenen Endes vorsteht, in einer Presspassung jeweils mit der ersten Klemmführungsnut (303) und der zweiten Klemmführungsnut (401) ist, der Filterhaltesitz (5) abgedichtet ist und in einer Spielpassung mit einem anderen Ende des transparenten Lichtleitersitzes (4) ist, und der Steuerungsschaltersitz (3) in einer Presspassung mit dem transparenten Lichtleitersitz (4) ist;
die zweiten Zigarettengaseinlasslöcher (502) sind jeweils mit dem ersten Zigarettengaseinlassloch (405) und dem Zigarettengaseinlasskanal (304) an dem Steuerungsschaltersitz (3) durch einen Spalt zwischen dem Filterhaltesitz (5) und dem transparenten Lichtleitersitz (4) verbunden;
eine Isolationsbefestigungshülse (6), wobei die Isolationsbefestigungshülse eine zylindrisch-artige Form aufweist und den Filterhaltesitz (5) in einer Presspassung von außen umhüllt;
eine Ladebasis (7), wobei die Ladebasis eine zylindrische Form hat, ein oberer Abschnitt der Ladebasis die Isolationsbefestigungshülse (6) in einer Presspassung von außen umhüllt, ein unterer Abschnitt der Ladebasis innerhalb eines Mundstückrohrs (1) eingeschoben ist, ein mittlerer Abschnitt der Ladebasis mit einer Vielzahl von Gasführungslöchern (71) versehen ist, die mit der Steuerungsschalter-Gaseinlassnut (403) und dem Steuerungsschalter-Gaseinlasskanal (302) verbunden sind;
Luft tritt von den Gasführungslöchern (71) in die Steuerungsschalter-Gaseinlassnut (403) und den Steuerungsschalter-Gaseinlasskanal (302) ein und tritt dann in den Steuerungsschalter (2) ein, um die elektronische Zigarette zu initialisieren;
Rauchgas tritt von den zweiten Zigarettengaseinlasslöchern (502) in den Spalt zwischen dem Filterhaltesitz (5) und dem transparenten Lichtleitersitz (4) ein, um in das erste Zigarettengaseinlassloch (405) einzutreten, und tritt dann in den Zigarettengaseinlasskanal (304) ein, und somit in das Mundstückrohr (1).

2. Die Vorrichtung mit dem getrennten Gaseinlass für Zigarettenset aus Anspruch 1, wobei der untere Abschnitt der Ladebasis (7) in einer Presspassung innerhalb des Mundstückrohrs (1) eingeschoben ist.

3. Die Vorrichtung mit dem getrennten Gaseinlass für Zigarettenset aus Anspruch 1, wobei der Zigarettengaseinlasskanal (304) mit dem Mundstückrohr (1) verbunden ist.

## Revendications

1. Dispositif avec une entrée de gaz séparée pour un ensemble de cigarette, comprenant :
un interrupteur de commande (2), dans lequel l'interrupteur de commande est un capteur de débit d'air cylindrique ayant deux extrémités, et l'air s'écoule entre les deux extrémités ;
un siège d'interrupteur de commande (3), dans lequel le siège d'interrupteur de commande est une structure en forme de cylindre avec deux extrémités, l'air s'écoule à travers chaque extrémité, un cylindre intérieur est un trou d'interrupteur de commande (301) fixé fermement à une paroi extérieure de l'interrupteur de commande (2), une paroi perpendiculaire à une extrémité du siège d'interrupteur de commande (3) est munie d'un canal d'entrée de gaz d'interrupteur de commande (302) en forme de U, qui relie un intérieur du cylindre à un extérieur du cylindre, et une paroi de l'extrémité est munie d'une rainure de paroi latérale extérieure (305), deux rainures sont prévues le long d'une direction axiale d'une paroi latérale extérieure du cylindre, à savoir respectivement une première rainure de guidage du serrage (303) et un canal d'entrée de gaz de cigarette (304) ;
un siège de guidage de lumière transparent (4), dans lequel le siège de guidage de lumière transparent est une structure en forme de cylindre fermé au centre, une deuxième rainure de guidage du serrage (401) est prévue le long d'une direction axiale d'un côté intérieur d'une paroi extérieure du cylindre, une paroi perpendiculaire à une extrémité du siège de guidage de lumière transparent (4) est munie d'une rainure d'entrée de gaz d'interrupteur de commande (403) en forme de U, qui relie un intérieur du cylindre à un extérieur du cylindre, et l'extrémité est munie d'un interrupteur de commande, qui correspond à une paroi faisant saillie (404) et protubérant vers l'extérieur et étroitement fixée à la rainure de paroi latérale extérieure (305), une partie centrale fermée au centre du cylindre est un bouchon à trou d'interrupteur de commande (402), un premier trou d'entrée de gaz de cigarette (405) relié au canal d'entrée de gaz de cigarette (304) est disposé entre le bouchon à trou d'interrupteur de commande (402) et la paroi de cylindre ;
un siège de retenue de filtre (5), dans lequel le siège de retenue de filtre est une structure en forme de cylindre avec une extrémité fermée, une pluralité de petits trous prévus à l'extrémité fermée sont des trous d'entrée de gaz de cigarette secondaires (502), une colonne de guidage du serrage (501) protubérant le long d'une direction axiale de l'extrémité fermée correspond en interférant respectivement à la première rainure de guidage du serrage (303) et à la deuxième rainure de guidage du serrage (401), le siège de retenue de filtre (5) est adapté et ajusté avec jeu à une autre extrémité du siège de guidage de lumière transparent (4) et le siège d'interrupteur de commande (3) est en correspondance d'interférence avec le siège de guidage de lumière transparent (4) ;
les trous d'entrée de gaz de cigarette secondaires (502) sont chacun reliés au premier trou d'entrée de gaz de cigarette et au canal d'entrée de gaz de cigarette (304) au siège d'interrupteur de commande (3) par une fente entre le siège de retenue de filtre (5) et le siège de guidage de lumière transparent (4) ; un manchon de fixation d'isolation (6), dans lequel le manchon de fixation d'isolation est en forme cylindrique et est disposé à l'extérieur du siège de retenue de filtre (5) en correspondance d'interférence ;
une base de chargement (7), dans lequel la base de chargement est en forme cylindrique, une partie supérieure de la base de chargement est manchonnée à l'extérieur du manchon de fixation d'isolation(6) en correspondance d'interférence, une partie inférieure de la base de chargement est manchonnée à l'intérieur d'un tube d'embouchure (1), une partie médiane de la base de chargement est munie d'une pluralité de trous de guidage de gaz (71) reliés à la rainure d'entrée de gaz d'interrupteur de commande (403) et au canal d'entrée de gaz d'interrupteur de commande (302) ;
l'air entre dans la rainure d'entrée de gaz d'interrupteur de commande (403) et dans le canal d'entrée de gaz d'interrupteur de commande (302) à partir de trous de guidage de gaz (71) et entre ensuite dans l'interrupteur de commande (2) pour initier la cigarette électronique ;
du gaz de fumée entre dans la fente entre le siège de retenue de filtre (5) et le siège de guidage de lumière transparent (4) à partir des trous d'entrée de gaz de cigarette secondaires (502) pour entrer dans le premier trou d'entrée de gaz de cigarette (405) et entre ensuite dans le canal d'entrée de gaz de cigarette (304) et ainsi dans le tube d'embouchure (1).

2. Dispositif avec une entrée de gaz séparée pour un ensemble de cigarette selon la revendication 1, dans lequel la partie inférieure de la base de chargement (7) est manchonnée dans le tube d'embouchure (1) en correspondance d'interférence.

3. Dispositif avec une entrée de gaz séparée pour un ensemble de cigarette selon la revendication 1, dans lequel le canal d'entrée de gaz de cigarette (304) est relié au tube d'embouchure (1).
